# EUROPEAN PATENT SPECIFICATION

(11) **EP 3 902 493 B1**
(45) Date of publication and mention of the grant of the patent: **28.12.2022**
(21) Application number: 20727773.2
(22) Date of filing: 01.05.2020
(51) Int. Cl.: A61B 18/14, A61B 18/00

(54) **ELECTRODE ASSEMBLY INCLUDING INNER AND OUTER BASKETS AND METHODS OF FORMING SAME**
ELEKTRODENANORDNUNG MIT INNEREN UND ÄUSSEREN KÖRBEN UND VERFAHREN ZU DEREN HERSTELLUNG
ENSEMBLE ÉLECTRODE COMPRENANT DES PANIERS INTERNE ET EXTERNE ET LEURS PROCÉDÉS DE FORMATION

(30) Priority: 03.05.2019 US 201962842654 P
(43) Date of publication of application: 03.11.2021
(73) Proprietor: St. Jude Medical, Cardiology Division, Inc., St. Paul MN 55117 (US)
(72) Inventor: DALE, Ted, Corcoran, Minnesota 55340 (US); TEGG, Troy, Elk River, Minnesota 55330 (US); MANDA, Rishi, Stillwater, Minnesota 55082 (US)
(74) Representative: Kramer Barske Schmidtchen Patentanwälte PartG mbB
(86) International application number: PCT/US2020/031004
(87) International publication number: WO 2020/227086

(56) References cited:
- WO-A1-99/23961
- US-A1- 2013 231 659

## Description

### FIELD OF THE DISCLOSURE

This disclosure relates to ablating tissue, and more particularly, this disclosure relates to an electrode assembly including inner and outer baskets for ablating tissue.

### BACKGROUND

Catheter systems are well known in the art for use in medical procedures, such as diagnostic, therapeutic and ablative procedures. Typical catheter systems generally include an elongate catheter extending from a handle. A physician manipulates the catheter through the patient's vasculature to an intended site within the patient. The catheter typically carries one or more working components, such as electrodes and thermocouples, or other diagnostic, therapeutic, or ablative devices for carrying out the procedures. One or more controls or actuators may be provided on the handle for selectively adjusting one or more characteristics of the working components. Further, methods and apparatuses for modulating nerves through the walls of blood vessels are known from US 2013/0231659 A1

One particular example of a multi-electrode catheter system is an ablative catheter system in which the working component is a multi-electrode assembly carried at the distal end of a flexible catheter. A control wire generally extends within the catheter from the multi-electrode assembly to the handle to operatively connect the multi-electrode assembly to an actuator on the handle. Manipulating the actuator acts on the control wire to configure the multi-electrode assembly into a desired configuration for carrying out the ablative procedure. For example, in one such ablative catheter system made by Abbott Laboratories under the trade name EnligHTN, the multi-electrode assembly is an electrode assembly in the general form of an electrode basket. The electrode basket generally includes a number of struts, wherein each strut may include one or two electrodes. In at least some known catheter systems, however, the electrode basket is relatively long, and different struts may expand and collapse at different times, and by different amounts. The electrodes may not contact the tissue as desired, which may interfere with ablation processes.

Moreover, for "one-shot" ablation procedures, which reduce the number of ablations needed and therefore reduce an overall procedure duration, a complex current is applied to the multi-electrode ablation assembly. The spacing between electrodes on the multi-electrode assembly - specifically between cathode electrodes and anode electrodes, or between the multi-electrode assembly and an external "patch" electrode - may induce skeletal "jumping," or spasms in the patient tissue, during such one-shot ablation procedures.

### BRIEF SUMMARY OF THE DISCLOSURE

The invention is defined by independent claims 1 and 10. Preferred embodiments are defined in the dependent claims.

The foregoing and other aspects, features, details, utilities and advantages of the present disclosure will be apparent from reading the following description and claims, and from reviewing the accompanying drawings.

### BRIEF DESCRIPTION OF THE DRAWINGS

Figure 1 is a perspective view of one embodiment of a catheter system including a handle, a catheter, and an electrode assembly.
Figures 2-4 depict one example embodiment of the electrode assembly shown in Figure 1, including an inner electrode basket and an outer electrode basket.
Figure 5 is a perspective view of a first embodiment of an inner electrode basket.
Figure 6 is a perspective view of a first embodiment of an outer electrode basket.
Figure 7 is a side view of a second embodiment of an inner electrode basket.
Figure 8 is a side view of a second embodiment of an outer electrode basket.
Figure 9 is a perspective view of the electrode assembly of Figure 2 illustrating the electrode assembly during a tissue ablation procedure.
Figures 10 and 11 illustrate the electrode assembly shown in Figures 2-4 including electrically insulating material to insulate the inner electrode basket from the outer electrode basket.
Figure 12 is another perspective view of the electrode assembly shown in Figures 2-4 with the outer electrode basket depicted in phantom.
Figure 13 is a side view of another embodiment of an electrode assembly suitable for use with the catheter system of Figure 1.
Figures 14-16 illustrate the electrode assembly of Figures 2-4 being deployed from the catheter shown in Figure 1, from a collapsed configuration to an expanded configuration.
Figures 17 and 18 illustrate an alternative embodiment of an electrode assembly.
Figure 19 is a simplified process diagram for a method of forming an electrode assembly.
Figure 20 is a simplified process diagram for a method of performing an ablation procedure using an electrode assembly.

Corresponding reference characters indicate corresponding parts throughout the several views of the drawings.

### DETAILED DESCRIPTION OF THE DISCLOSURE

The disclosure provides systems and methods for electrode assemblies including an inner basket and an outer basket. Each basket includes a plurality of struts configured to be energized. One of the inner and the outer baskets functions as an anode or negative (-) electrode, and the other of the inner and outer baskets functions as a cathode or positive (+) electrode. When the electrode assembly is constructed, the struts of the inner and outer basket alternate with one another (e.g., in a + - + - pattern). Accordingly, when the electrode assembly is fully energized and advanced into contact with tissue, the electrode assembly functions as a plurality of bipolar electrode pairs (e.g., each pair of adjacent (+ -) struts), and provides a full 360° circumferential ablation of the contacted tissue.

The electrode assembly described herein is particularly suitable for "one-shot" ablation procedures, also referred to as "irreversible electroporation (IRE)" procedures, in which it is desirable to complete the ablation procedure in a single ablation event (or in very few ablation events). Specifically, because the electrode assembly enables 360°, fully circumferential tissue contact, an entire circumferential ablation can be completed in a single ablation event (or in very few ablation events).

The electrode assembly may also reduce the occurrence and/or magnitude of skeletal jumping in the patient's tissue. Specifically, the spacing between electrodes is reduced by effectively doubling the number of struts (and therefore the number of adjacent electrodes). In addition, different currents may be applied to the electrode assembly to reduce the occurrence and/or magnitude of skeletal jumping in the patient's tissue.

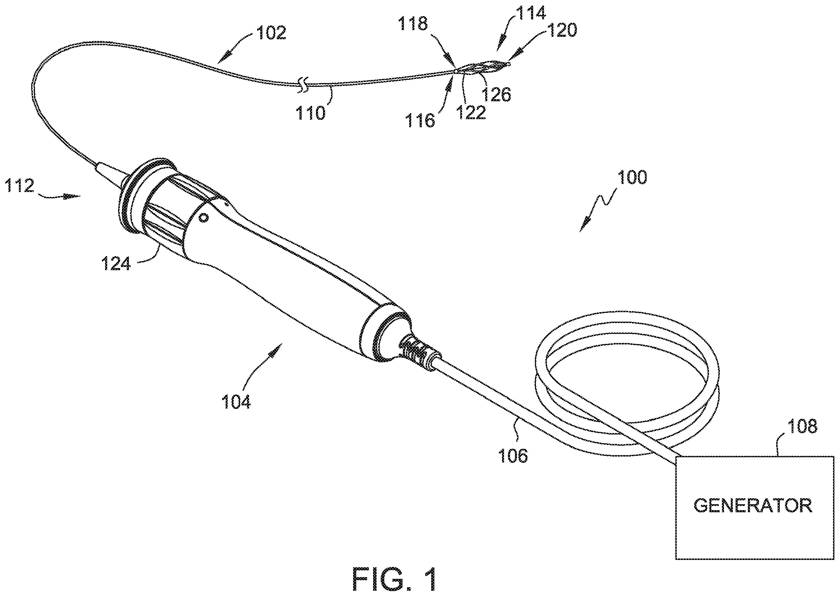

Referring now to the drawings wherein like reference numerals are used to identify identical components in the various views, Figure 1 illustrates a catheter system 100. Catheter system 100 includes a flexible catheter 102, a handle 104 to which flexible catheter 102 is connected, and a conductor assembly 106 for electrically connecting catheter system 100 to a suitable power supply, such as a generator 108.

As one example, catheter system 100 illustrated and described herein is suitably constructed for use as an ablation system, such as a renal or heart ablation system, for ablation of tissue. General operation of a renal denervation system is known to those of skill in the art and is not described further herein except to the extent necessary to describe the present embodiments. In the exemplary embodiment, "tissue" includes heart or cardiac tissue within a human body. It should be understood, however, that catheter system 100 may find application in connection with a variety of other tissues within human and non-human bodies, and therefore, the present disclosure is not meant to be limited to the use of catheter system 100 in connection with only cardiac tissue and/or human bodies.

It is also understood that catheter system 100 may be used for any other suitable treatment or purpose without departing from the scope of this disclosure, such as mapping procedures. In such embodiments, catheter system 100 may include a display device (not shown) for visualization, navigation, and/or mapping of internal body structures within the patient.

Additionally, while catheter system 100 is illustrated and described herein as including flexible catheter 102, such as, for example, an electrophysiology catheter, catheter system 100 may further include other components used, for example, to guide flexible catheter 102 into the patient-such as, without limitation, a relatively more rigid guide catheter or sheath (not shown) or guide wire (not shown).

Flexible catheter 102 includes an elongate, flexible hollow catheter shaft 110 connected to handle 104 at or near a proximal or rear end 112 of catheter shaft 110, and an electrode assembly 114 disposed at or near a distal or front end 116 of catheter shaft 110. Electrode assembly 114 includes a proximal end 118 and a distal end 120. As used herein, the terms proximal and front, and distal and rear, are used with reference to the orientation of catheter system 100 illustrated in the various drawings and for the purpose of describing the various embodiments set forth herein, and are not intended as limiting the catheter system and related components to having any particular orientation upon assembly or during operation thereof. In particular, the terms proximal and rear refer to a longitudinal position that is relatively nearer to handle 104 while the terms distal and front refer to a longitudinal position that is relatively farther from handle 104.

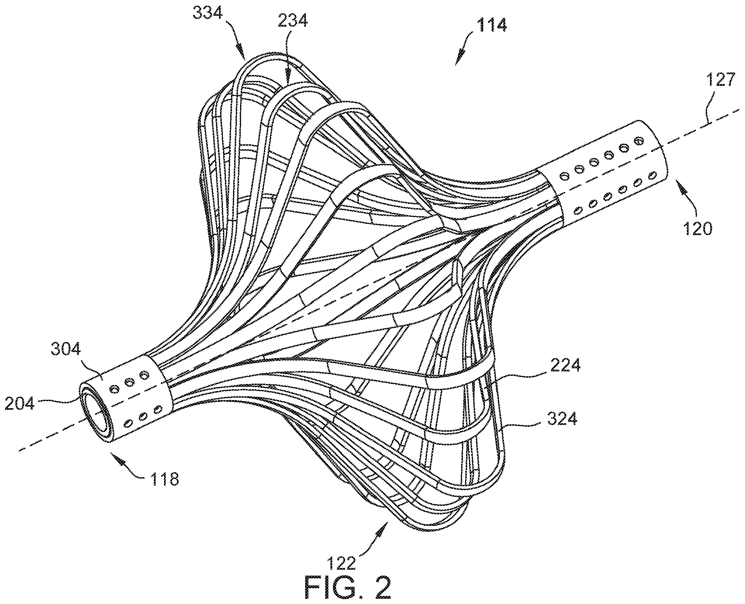

The illustrated electrode assembly 114 is in the form of what may be referred to as an electrode basket and includes struts 122. Electrode assembly 114 is configurable between a collapsed configuration (see Figure 1) for maneuvering and positioning the electrode assembly in the patient's vasculature, and an expanded configuration (see Figures 2-4) for operation of the electrode assembly to perform a desired procedure, such as an ablation procedure. Electrode assembly 114 may be delivered to a target site within the patient in the collapsed configuration within catheter shaft 110. Handle 104 includes an actuator 124 operatively connected to electrode assembly 114 for selectively configuring electrode assembly 114 between its collapsed and expanded configurations and/or for maneuvering electrode assembly 114 proximally and distally within the patient's vasculature. Although actuator 124 is embodied as a rotation actuator in the illustrated embodiment, actuator 124 may include, for example, one or more of a slide actuator, a push button, a lever, and/or combinations thereof. In some embodiments, electrode assembly 114 may be selectively adjustable (e.g., using actuator 124) between an infinite number of configurations (e.g., degrees of expansion) between its collapsed and expanded configurations. Handle 104 provides a location for a user to hold catheter 102 and provides means for steering or guiding shaft 110 within the patient's body. In some embodiments, control of catheter 102 may be automated such as by being robotically driven or controlled, or driven and controlled by a magnetic-based guidance system. Accordingly, catheters controlled either manually or automatically are both within the scope of the present disclosure.

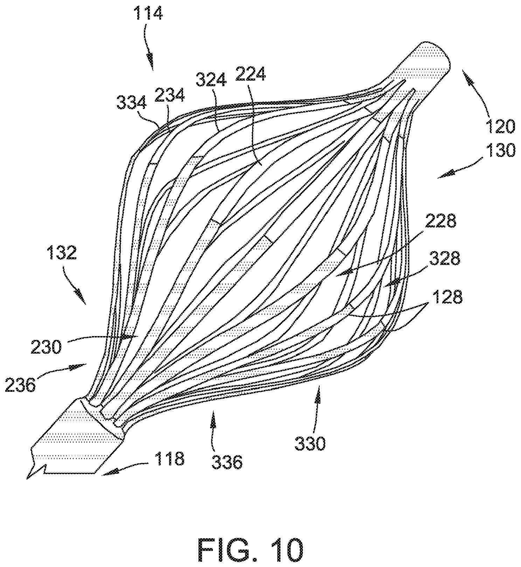

A control line, such as a suitable cable or pull wire 126 (shown in greater detail in Figures 10 and 11 and 14-16) extends from electrode assembly 114 within hollow catheter shaft 110 and into handle 104 for operative connection with actuator 124 to thereby operatively connect actuator 124 with electrode assembly 114. In some embodiments, two or more pull wires, cables, or other suitable control lines or tubes may be used for selectively configuring electrode assembly 114. It is also understood that control line 126 may be any suitable control line other than a pull wire, such as a cable, string, tie, compression member or other suitable control to operatively connect electrode assembly 114 to actuator 124. A suitable electrical wire bundle (not shown) also extends through hollow catheter shaft 110 from handle 104 to electrode assembly 114 to deliver power to, and receive feedback from, electrode assembly 114, as described further herein.

In some embodiments, catheter 102 and/or electrode assembly 114 may further include other conventional components such as, for example and without limitation, steering wires and actuators, irrigation lumens and ports, pressure sensors, contact sensors, temperature sensors, and/or positioning sensors.

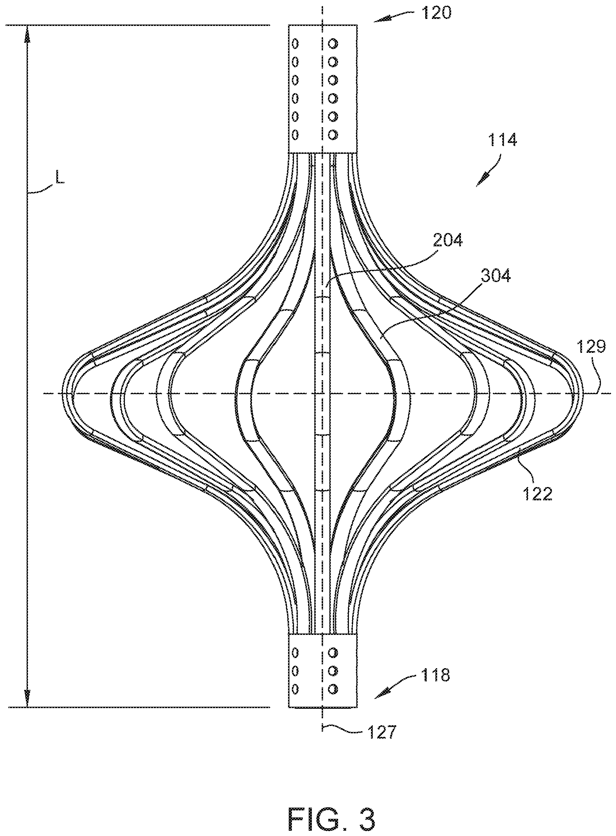

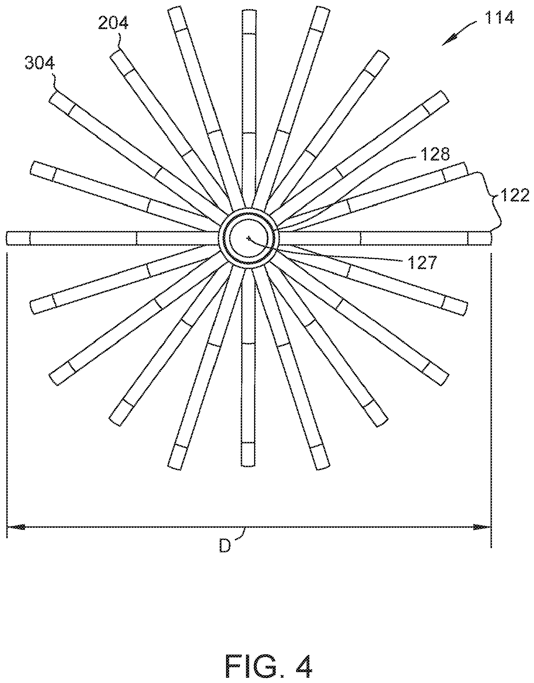

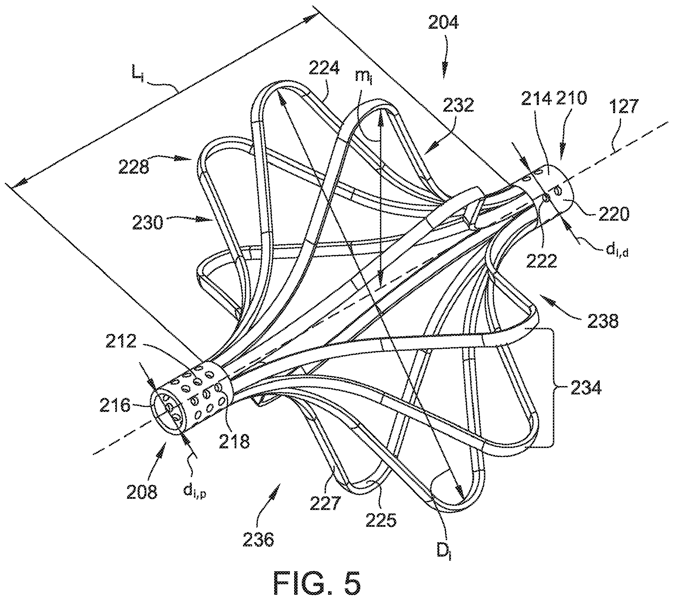

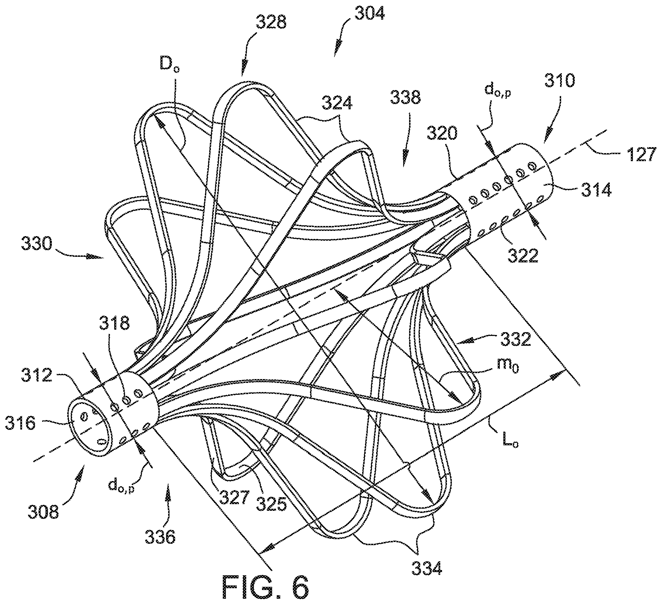

Turning now to Figure 2, a perspective view of an exemplary electrode assembly 114 is illustrated. Figures 3 and 4 depict a side view and a longitudinal view of electrode assembly 114 (i.e., a view taken along a longitudinal axis 127 of electrode assembly 114), respectively. Electrode assembly 114 extends from proximal end 118 to distal end 120 along longitudinal axis 127, and has an overall assembly length *L* and an overall assembly diameter *D.* Assembly length *L* and assembly diameter *D* vary as electrode assembly 114 is transitioned between its collapsed configuration and its expanded configuration. More specifically, assembly length *L* decreases and assembly diameter *D* increases as electrode assembly 114 is transitioned between its collapsed configuration and its expanded configuration. In the exemplary embodiment, electrode assembly 114 includes two electrode baskets, specifically, an inner basket 204 and an outer basket 304. Inner basket 204 is shown in greater detail in Figure 5, and outer basket 304 is shown in greater detail in Figure 6.

With reference to Figure 5, inner basket 204 extends from a proximal end 208 to a distal end 210. In the exemplary embodiment, proximal end 208 is generally defined by an annular or tubular proximal end portion or collar 212, and distal end 210 is generally defined by an annular or tubular distal end portion or collar 214. Annular proximal end portion 212 is an annular or tubular structure including a wall 216 that extends continuously about a circumference of proximal end 208 and has a diameter *d_{i,p}.* Wall 216 has a plurality of through-holes 218 defined therein. In the illustrated embodiment, through-holes 218 are arranged in a regular pattern about wall 216, in a plurality of rows and columns. In other embodiments, wall 216 includes any number of through-holes 218 arranged in any suitable regular and/or irregular pattern.

Likewise, annular distal end portion 214 is an annular or tubular structure including a wall 220 that extends continuously about a circumference of distal end 210 and has a diameter *d_{i,d}.* Diameter *d_{i,d}* is the same as diameter *d_{i,p},* in the exemplary embodiment. Wall 220 has a plurality of through-holes 222 defined therein. In the illustrated embodiment, through-holes 222 are arranged in a regular pattern about wall 220, in a plurality of rows and columns. In other embodiments, wall 220 includes any number of through-holes 222 arranged in any suitable regular and/or irregular pattern.

When inner basket 204 is coupled to outer basket 304 to form electrode assembly 114, as described in greater detail herein, proximal end 208 of inner basket 204 (e.g., annular proximal end portion 212) partially defines proximal end 118 of electrode assembly 114. Likewise, when inner basket 204 is coupled to outer basket 304 to form electrode assembly 114, distal end 210 of inner basket 204 (e.g., annular distal end portion 214) partially defines distal end 120 of electrode assembly 114.

Inner basket 204 further includes a plurality of struts 224 that extend from proximal end 208 to distal end 210 of inner basket 204. Specifically, struts 224 have a strut length *Lᵢ* defined between annular proximal end portion 212 and annular distal end portion 214. Inner basket 204 may include any number of struts 224, including at least 8 struts, or at least 10 struts, or at least 12 struts. Struts 224 each have an inner surface 225 (which may also refer generally to an inner surface of inner basket 204) and an outer surface 227 (which may also refer generally to an outer surface of inner basket 204). As described further herein, struts 224 are formed from a shape memory material such that struts 224 tend to return to a particular shape or profile in the absence of any force thereon. In the illustrated embodiment, struts 224 have a generally arcuate and continuous profile that is symmetrical about longitudinal axis 127 and across a plane (not shown) bisecting inner basket 204. In particular, the profile of each strut 224 is the same. In alternative embodiments, the profile of struts 224 may vary (e.g., each strut 224 may have a different profile, adjacent struts 224 may alternate between two profiles, etc.).

In the expanded configuration, inner basket 204 has a diameter *Dᵢ.* Additionally, in the exemplary embodiment, struts 224 are equally spaced from one another. More specifically, a distance between each pair of adjacent struts 224 is the same. Each strut includes a central portion 228, a proximal portion 230 that extends between central portion 228 and annular proximal end portion 212, and a distal portion 232 that extends between central portion 228 and annular distal end portion 214. Proximal portion 230, central portion 228, and distal portion 232 extend continuously from one another, such that strut 224 is continuously curvilinear. In an alternative embodiment, proximal portion 230, central portion 228, and/or distal portion 232 are connected to one another via a hinge (e.g., a living hinge).

In the illustrated embodiment, the profile of struts 224 - specifically, the profile of struts 224 within central portion 228 - defines a radial maximum or outermost point 234 of each strut 224. In the illustrated embodiment, each maximum 234 (of each strut 224) is the same distance *mᵢ* from longitudinal axis 127 when inner basket 204 is in an expanded configuration (corresponding to the expanded configuration of electrode assembly 114). Moreover, maximum 234 is located at a center of strut 224, such that struts 224 are symmetrical across a plane bisecting inner basket 204. Proximal and distal portions 230, 232 extend symmetrically inwardly from central portion 228 as they extend towards proximal and distal ends 208, 210, respectively. Accordingly, inner basket 204 includes a proximal basket half 236 including proximal portions 230 and a proximal section of central portions 228 proximal to maximum 234, as well as a distal basket half 238 including distal portions 232 and a distal section of central portions 228 distal from maximum 234.

In alternative embodiments, struts 224 may include more than one maximum and/or may include a maximum located elsewhere along strut 224 (e.g., within proximal portion 230 or distal portion 232, or at another, non-center position within central portion 228). In such embodiments, proximal basket half 236 may therefore represent less or more than a "half' of inner basket 204, and distal basket half 238 may represent less or more than a "half' of inner basket 204. More particularly, proximal basket half 236 may represent the portion of inner basket 204 proximal to the proximal-most maximum, and distal basket half 238 may represent the portion of inner basket 204 distal to the distal-most maximum (where the proximal-most and distal-most maxima may be the same or different maxima).

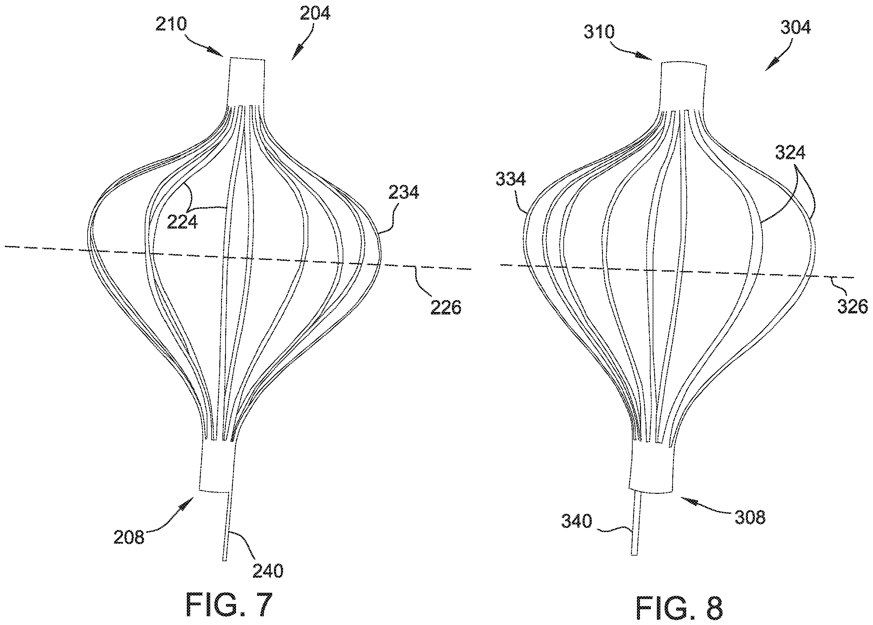

Another embodiment of inner basket 204 is shown in Figure 7. In the illustrated embodiment, maximum 234 is not at a center of each strut 224, but rather is distally offset, and inner basket 204 is not symmetrical across a plane 226 bisecting inner basket 204. In addition, inner basket 204 includes a tab 240 extending from proximal end 208. Tab 240 enables electrical connection between inner basket 204 and electrical cables (not shown) that provide power to inner basket 204 (e.g., from generator 108, shown in Figure 1). Inner basket 204 may include only one tab 240, a pair of tabs 240, or additional/alternative tabs and/or suitable electrical connection points.

To form inner basket 204, in one embodiment, a tube (not shown) of suitable material is provided. In the exemplary embodiment, the tube is formed of an electrically conductive shape memory material, such that inner basket 204 is electrically conductive and may be treated to retain a desired shape. Suitable materials may include, for example and without limitation, copper-aluminum-nickel alloys, nickel-titanium (NiTi) alloys, nitinol, alloys including zinc, copper, gold, and/or iron, polymers including any of the above materials, and/or combinations thereof. The tube is cut to a desired length, such as an overall length of inner basket 204 between proximal end 208 and distal end 210. In some embodiments, the length of the tube further includes a length of one or more tabs 240. Alternatively, tabs 240 may be coupled to the tube (e.g., by welding, bonding using a conductive adhesive, etc.).

Through-holes 218 and 222 are formed in the tube, for example, via laser-cutting, die-cutting, and/or any other suitable method. Subsequently, the tube is slit longitudinally (e.g., parallel to a longitudinal axis thereof) to form struts 224, for example, via laser-cutting, die-cutting, and/or any other suitable method. This step also defines annular proximal end portion 212 and annular distal end portion 214, specifically, as the portions of the tube that are not slit. In some embodiments, the tube is slit before the through-holes 218, 222 are formed.

Thereafter, the tube is positioned about a mandrel and heat-set to define the shape or profile of struts 224. For example, the mandrel has an outer surface complementary to the desired shape of struts 224 and/or the desired shape of inner basket 204 in the expanded configuration. The resulting inner basket 204 is a unitary, single-piece basket having struts 224 that retain a desired shape or profile (such as that shown in Figure 5 and/or Figure 7) extending between annular proximal end portion 212 and annular distal end portion 214, having through-holes 218 and 222, respectively, defined therein.

Inner basket 204 may be formed using an alternative method, such as forming struts 224 and attaching annular proximal end portion 212 and annular distal end portion 214 thereto (e.g., via welding, bonding using a conductive adhesive, etc.).

Turning now to Figure 6, in the illustrated embodiment, outer basket 304 is substantially similar to inner basket 204, and similar components common to inner basket 204 and outer basket 304 will be referred to with reference numerals beginning with "3". Outer basket 304 extends from a proximal end 308 to a distal end 310. In the exemplary embodiment, proximal end 308 is generally defined by an annular or tubular proximal end portion or collar 312, and distal end 310 is generally defined by an annular or tubular distal end portion or collar 314. Annular proximal end portion 312 is an annular or tubular structure including a wall 316 that extends continuously about a circumference of proximal end 308 and has a diameter *d_{o,p}.* Wall 316 has a plurality of through-holes 318 defined therein. In the illustrated embodiment, through-holes 318 are arranged in a regular pattern about wall 316, in a plurality of rows and columns. In other embodiments, wall 316 includes any number of through-holes 318 arranged in any suitable regular and/or irregular pattern.

Likewise, annular distal end portion 314 is an annular or tubular structure including a wall 320 that extends continuously about a circumference of distal end 310 and has a diameter *d_{o,d}.* Diameter *d_{o,d}* is the same as diameter *d_{o,p},* in the exemplary embodiment. Wall 320 has a plurality of through-holes 322 defined therein. In the illustrated embodiment, through-holes 322 are arranged in a regular pattern about wall 320, in a plurality of rows and columns. In other embodiments, wall 320 includes any number of through-holes 322 arranged in any suitable regular and/or irregular pattern.

When inner basket 204 is coupled to outer basket 304 to form electrode assembly 114, as described in greater detail herein, proximal end 308 of outer basket 304 (e.g., annular proximal end portion 312) partially defines proximal end 118 of electrode assembly 114. Likewise, when inner basket 204 is coupled to outer basket 304 to form electrode assembly 114, distal end 310 of outer basket 304 (e.g., annular distal end portion 314) partially defines distal end 120 of electrode assembly 114.

Outer basket 304 further includes a plurality of struts 324 that extend from proximal end 308 to distal end 310 of outer basket 304. Specifically, struts 324 have a strut length *Lₒ* defined between annular proximal end portion 312 and distal proximal end portion 314. Outer basket 304 may include any number of struts 324, including at least 8 struts, or at least 10 struts, or at least 12 struts. In the exemplary embodiment outer basket 304 includes the same number of struts 324 as the number of struts 224 of inner basket 204. Struts 324 each have an inner surface 325 (which may also refer generally to an inner surface of outer basket 304) and an outer surface 327 (which may also refer generally to an outer surface of outer basket 304). As described further herein, struts 324 are formed from a shape memory material such that struts 324 tend to return to a particular shape or profile in the absence of any force thereon. In the illustrated embodiment, struts 324 have a generally arcuate and continuous profile that is symmetrical about longitudinal axis 127 and across a plane (not shown) bisecting outer basket 304. In particular, the profile of each strut 324 is the same. In alternative embodiments, the profile of struts 324 may vary (e.g., each strut 224 may have a different profile, adjacent struts 224 may alternate between two profiles, etc.).

In the expanded configuration, outer basket 304 has a diameter *Dₒ* Additionally, in the exemplary embodiment, struts 324 are equally spaced from one another. More specifically, a distance between each pair of adjacent struts 324 is the same. Each strut includes a central portion 328, a proximal portion 330 that extends between central portion 328 and annular proximal end portion 312, and a distal portion 332 that extends between central portion 328 and annular distal end portion 314. Proximal portion 330, central portion 328, and distal portion 332 extend continuously from one another, such that strut 324 is continuously curvilinear. In an alternative embodiment, proximal portion 330, central portion 328, and/or distal portion 332 are connected to one another via a hinge (e.g., a living hinge).

In the illustrated embodiment, the profile of struts 324 - specifically, the profile of struts 324 within central portion 328 - defines a radial maximum or outermost point 334 of each strut 324. In the illustrated embodiment, each maximum 334 (of each strut 324) is the same distance *mₒ* from longitudinal axis 127 when inner outer basket 304 is in an expanded configuration (corresponding to the expanded configuration of electrode assembly 114). Moreover, maximum 334 is located at a center of strut 324, such that struts 324 are symmetrical across a plane bisecting outer basket 304. Proximal and distal portions 330, 332 extend symmetrically inwardly from central portion 328 as they extend towards proximal and distal ends 308, 310, respectively. Accordingly, outer basket 304 includes a proximal basket half 336 including proximal portions 330 and a proximal section of central portions 328 proximal to maximum 334, as well as a distal basket half 338 including distal portions 332 and a distal section of central portions 328 distal from maximum 334.

In alternative embodiments, struts 324 may include more than one maximum and/or may include a maximum located elsewhere along strut 324 (e.g., within proximal portion 330 or distal portion 332, or at another, non-center position within central portion 328). In such embodiments, proximal basket half 336 may therefore represent less or more than a "half' of outer basket 304, and distal basket half 338 may represent less or more than a "half' of outer basket 304. More particularly, proximal basket half 336 may represent the portion of outer basket 304 proximal to the proximal-most maximum, and distal basket half 338 may represent the portion of outer basket 304 distal to the distal-most maximum (where the proximal-most and distal-most maxima may be the same or different maxima).

Another embodiment of outer basket 304 is shown in Figure 8. In the illustrated embodiment, maximum 334 is not at a center of each strut 324, but rather is distally offset, and outer basket 304 is not symmetrical across a plane 326 bisecting outer basket 304. In addition, outer basket 304 includes a tab 340 extending from proximal end 308. Tab 340 enables electrical connection between outer basket 304 and electrical cables (not shown) that provide power to outer basket 304 (e.g., from generator 108, shown in Figure 1). Outer basket 304 may include only one tab 340, a pair of tabs 340, or additional/alternative tabs and/or suitable electrical connection points.

To form outer basket 304, in one embodiment, a tube (not shown) of suitable material is provided. In the exemplary embodiment, the tube is formed of an electrically conductive shape memory material, such that outer basket 304 is electrically conductive and may be treated to retain a desired shape. Suitable materials may include, for example and without limitation, copper-aluminum-nickel alloys, nickel-titanium (NiTi) alloys, nitinol, alloys including zinc, copper, gold, and/or iron, polymers including any of the above materials, and/or combinations thereof. The tube is cut to a desired length, such as an overall length of outer basket 304 between proximal end 308 and distal end 310. In some embodiments, the length of the tube further includes a length of one or more tabs 340. Alternatively, tabs 340 may be coupled to the tube (e.g., by welding, bonding using a conductive adhesive, etc.).

Through-holes 318 and 322 are formed in the tube, for example, via laser-cutting, die-cutting, and/or any other suitable method. Subsequently, the tube is slit longitudinally (e.g., parallel to a longitudinal axis thereof) to form struts 324, for example, via laser-cutting, die-cutting, and/or any other suitable method. This step also defines annular proximal end portion 312 and annular distal end portion 314, specifically, as the portions of the tube that are not slit. In some embodiments, the tube is slit before the through-holes 318, 322 are formed.

Thereafter, the tube is positioned about a mandrel and heat-set to define the shape or profile of struts 324. For example, the mandrel has an outer surface complementary to the desired shape of struts 324 and/or the desired shape of outer basket 304 in the expanded configuration. The resulting outer basket 304 is a unitary, single-piece basket having struts 324 that retain a desired shape or profile (such as that shown in Figure 6 and/or Figure 8) extending between annular proximal end portion 312 and annular distal end portion 314, having through-holes 318 and 322, respectively, defined therein.

Outer basket 304 may be formed using an alternative method, such as forming struts 324 and attaching annular proximal end portion 312 and annular distal end portion 314 thereto (e.g., via welding, bonding using a conductive adhesive, etc.).

To form electrode assembly 114, as shown in Figures 2-4, inner basket 204 (shown in Figure 5) is positioned within and is co-axial with outer basket 304 (shown in Figure 6). Specifically, proximal ends 208 and 308 of inner and outer baskets 204 and 204, respectively, are co-located to define proximal end 118 of electrode assembly 114. Likewise, distal ends 210 and 310 of inner and outer baskets 204 and 304, respectively, are co-located to define distal end 120 of electrode assembly 114.

For example, inner basket 204 is transitioned into its collapsed configuration (e.g., such that inner basket 204 is substantially tubular), and inner basket 204 is moved longitudinally through annular proximal end portion 312 and/or annular distal end portion 314 of outer basket 304. In the exemplary embodiment, diameters *d_{i,p}* and *d_{i,d}* of annular proximal end portion 212 and annular distal end portion 214, respectively, of inner basket 204 are smaller than diameters *d_{o,p}* and *d_{o,d}* of annular proximal end portion 312 and annular distal end portion 314, respectively, of outer basket 304, such that inner basket 204 fits through annular proximal end portion 312 and/or annular distal end portion 314 of outer basket 304. Put another way, proximal end portion 312 and annular distal end portion 314 of outer basket 304 are sized to receive annular proximal end portion 212 and annular distal end portion 214 of inner basket 204. Accordingly, when electrode assembly 114 is formed from inner basket 204 and outer basket 304, inner basket 204 and outer basket 304 are coaxial with one another.

Inner basket 204 is oriented with respect to outer basket 304 (e.g., by rotating or angularly displacing inner basket 204 relative to outer basket 304) such that struts 224 of inner basket 204 alternate with and are equally spaced from struts 324 of outer basket 304. Accordingly, electrode assembly 114 includes struts 224 alternating with struts 324 about a circumference of the electrode assembly 114. Struts 224 are equidistant from adjacent struts 324. To facilitate spacing and positioning of struts 224 relative to adjacent struts 324, inner basket 204 and/or outer basket 304 may include one or more alignment features. In some embodiments, through-holes 218, 222 and through-holes 318, 322 are the alignment feature. Specifically, through-holes 218 are arranged to align with through-holes 318, and through-holes 222 are arranged to align with through-holes 322, when inner basket 204 is properly angularly offset from outer basket 304 (i.e., such that struts 224 are equidistant from adjacent struts 324). Additionally or alternatively, the alignment feature may include a tab on one of inner basket 204 and outer basket 304 and a slot sized and located to receive the tab, on the other of inner basket 204 and outer basket 304, when inner basket 204 is properly angularly offset from outer basket 304.

In the exemplary embodiment, inner basket 204 functions as one of a cathode or negative (-) electrode and an anode or positive (+) electrode of electrode assembly 114, and outer basket 304 functions as the other of the cathode and anode of electrode assembly 114. Specifically, inner basket 204 is electrically coupled to generator 108 to receive a cathode or anode signal, and outer basket 304 is electrically coupled to generator 108 to receive the other of the cathode or anode signal. In one exemplary embodiment, inner basket 204 is the anode, and outer basket 304 is the cathode.

Accordingly, the alternating pattern of struts 224 and 324 corresponds to an alternating pattern of charge (+/-/+/-) about the circumference of electrode assembly 114. Specifically, energized struts 224 and 324 define pairs of adjacent, opposite-charge electrodes (e.g., a negative (-) strut 224 adjacent to a positive (+) strut 324). When electrode assembly 114 is energized and advanced into contact with tissue, electrode assembly 114 generates a circumferential ablation pattern between each pair of adjacent struts 224, 324, thereby creating a 360° ablation pattern in a single ablation event (e.g., between an initial contact of electrode assembly 114 and the tissue, and the removal of the electrode assembly 114 from the tissue or the termination of the energization of electrode assembly 114).

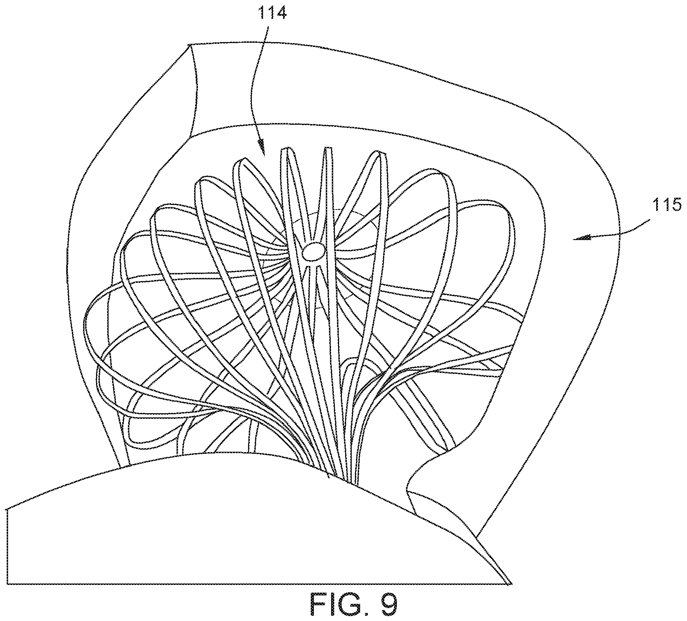

Figure 9 depicts electrode assembly 114 being advanced into contact with exemplary tissue 115. As shown, electrode assembly 114 is particularly suitable for complete, 360° contact with tissue 115. When energized, electrode assembly 114 functions as a plurality of electrode pairs between adjacent struts 224, 324, to generate an ablation pattern (not shown) about an entirety of the outer surface thereof that is in contact with tissue 115. Accordingly, struts 224, 324 may be referred to generally as "electrodes" or "ablation electrodes" of electrode assembly 114.

In the illustrated embodiment of Figures 2-4, electrode assembly 114 has diameter *D* that corresponds generally to diameter *Dₒ* of outer basket 304. In one embodiment, diameter *Dᵢ* is slightly less than *Dₒ*, by an amount corresponding to the difference between *d_{i,p}* and *d_{o,p},* (e.g., the difference in diameter between the tubes used to form inner basket 204 and outer basket 304). In another embodiment, *Dᵢ* is substantially the same as *Dₒ*. For example, struts 224 may be slightly longer than struts 324 and may be particularly heat-set such that maxima 234 are circumferentially aligned with maxima 334. Diameter *Dᵢ* and diameter *Dₒ* are generally sufficiently similar such that, when electrode assembly 114 is advanced into contact with tissue 115, struts 224 and 324 both sufficiently contact tissue 115 to ablate tissue 115.

In addition, electrode assembly 114 is substantially symmetrical about longitudinal axis 127 and across a plane 129 bisecting electrode assembly 114. Maxima 234 and maxima 334 are located at the same longitudinal or axial position on struts 224, 324 at plane 129, or at the center of struts 224, 324.

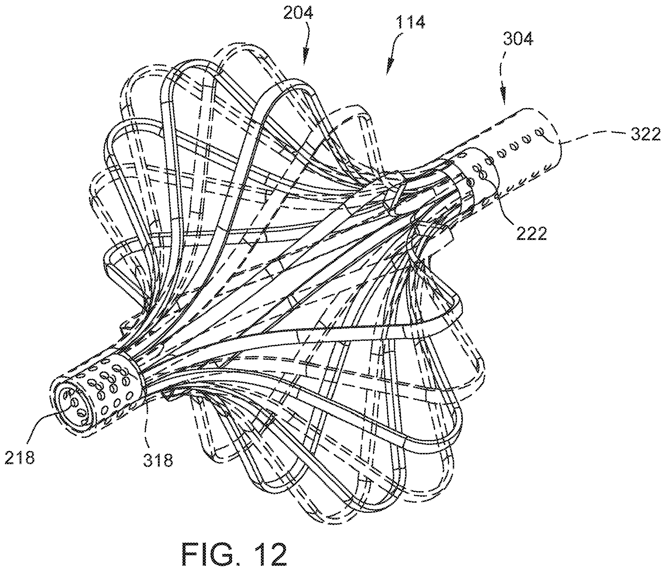

In the exemplary embodiment, to prevent electrical shorts between inner basket 204 and outer basket 304, inner basket 204 is electrically insulated from outer basket 304. Specifically, as electrode assembly 114 is formed, an electrically insulating material 128 (e.g., a dielectric coating, shown in Figures 4, 10 and 11) is provided between inner basket 204 and outer basket 304. In one embodiment, electrically insulating material 128 is re-flowed between inner basket 204 and outer basket 304, via through-holes 218, 222, 318, and/or 322. More particularly, insulating material 128 is reflowed (e.g., via through-holes 218 and/or 318) between annular proximal end portion 212 and annular proximal end portion 312, to prevent electrical contact therebetween. In addition, electrically insulating material 128 is reflowed (e.g., via through-holes 222 and/or 322) between annular distal end portion 214 and annular distal end portion 314, to prevent electrical contact therebetween. In one embodiment, through-holes 218 and 318 may not be directly aligned and/or through-holes 222 and 322 may not be directly aligned, to improve reflow of electrically insulating material 128 between annular proximal end portions 212 and 312 and/or annular distal end portions 214 and 314 (as illustrated in Figure 12, in which outer basket 304 is shown in phantom). In the exemplary embodiment, electrically insulating material 128 also facilitates adhering inner basket 204 to outer basket 304, to prevent angular displacement of inner basket 204 with respect to outer basket 304. Electrically insulating material 128 may be reflowed or otherwise provided on the sections of struts 224, 324 adjacent to annular end portions 212, 214, 312, 314, which may be in such close proximity to each other that electrical shorts could occur therebetween.

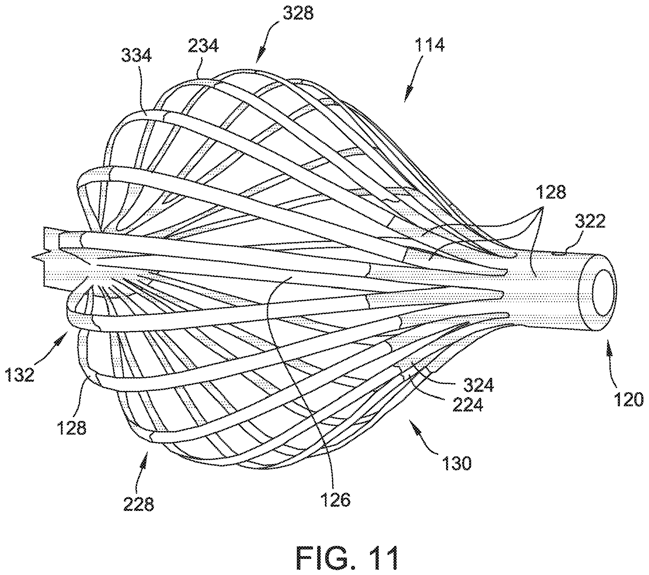

In addition, insulating material 128 is provided over inner and/or outer surface(s) 225, 227, 325, 327 of struts 224 and/or 324 to define a conductive portion 130 and a non-conductive portion 132 of electrode assembly 114 (shown in Figure 10). In the illustrated embodiment of Figures 10 and 11, proximal portions 230 of struts 224 and proximal portions 330 of struts 324 are coated in insulating material 128. Specifically, struts 224 and 324 are coated in insulating material 128 until just below (e.g., proximal of) maxima 234, 334 thereof. Accordingly, non-conductive portion 132 of electrode assembly 114 includes the portion(s) of struts 224, 324 coated in insulating material 128, including proximal portions 230, 330 (and, in some embodiments, at least a section of central portions 228, 328 proximal of maxima 234, 334). For example, non-conductive portion 132 of electrode assembly 114 includes proximal basket half 236 of inner basket and proximal basket half 336 of outer basket 304. Conductive portion 130 of electrode assembly 114 includes the portion(s) of struts 224, 324 that are free of insulating material 128, including distal portions 232, 332 (and, in some embodiments, maxima 234, 334 and at least a second of central portions 228, 328 distal of maxima 234, 334). For example, conductive portion 130 includes distal basket half 238 of inner basket 204 and distal basket half 338 of outer basket 304.

"Conductive portion 130" may refer to any of (i) the conductive (i.e., uninsulated) portion(s) of inner basket 204 and struts 224, (ii) the conductive (i.e., uninsulated) portion(s) of outer basket 304 and struts 324, and/or (iii) the conductive (i.e., uninsulated) portion(s) of formed electrode assembly 114. Likewise, "non-conductive portion 132" may refer to any of (i) the non-conductive (i.e., insulated) portion(s) of inner basket 204 and struts 224, (ii) the non-conductive (i.e., insulated) portion(s) of outer basket 304 and struts 324, and/or (iii) the non-conductive (i.e., insulated) portion(s) of formed electrode assembly 114.

When electrode assembly 114 is advanced into contact with tissue (e.g., tissue 115, shown in Figure 9) and energized, conductive portion 130 of electrode assembly 114 transmits ablative energy into the tissue, whereas non-conductive portion 132 is insulated from the tissue. Accordingly, conductive portions 130 of struts 224 and conductive portions 130 of struts 324 may be referred to as the "electrodes" and/or "ablation electrodes" of electrode assembly 114.

Varying configurations (e.g., locations, sizes, and/or orientations) of conductive portion 130 and non-conductive portion 132 are contemplated within the scope of the present disclosure. The specific configuration can be selected and created by selectively providing insulating material 128 on electrode assembly 114. Insulating material 128 may be deposited on inner basket 204, outer basket 304, and/or the formed electrode assembly 114. Additionally or alternatively, inner basket 204, outer basket 304, and/or the formed electrode assembly 114 may be dipped into insulating material 128 to coat struts 224, 324 (as well as, in some embodiments, proximal end(s) 208, 308, 118 and/or distal end(s) 210, 310, 120).

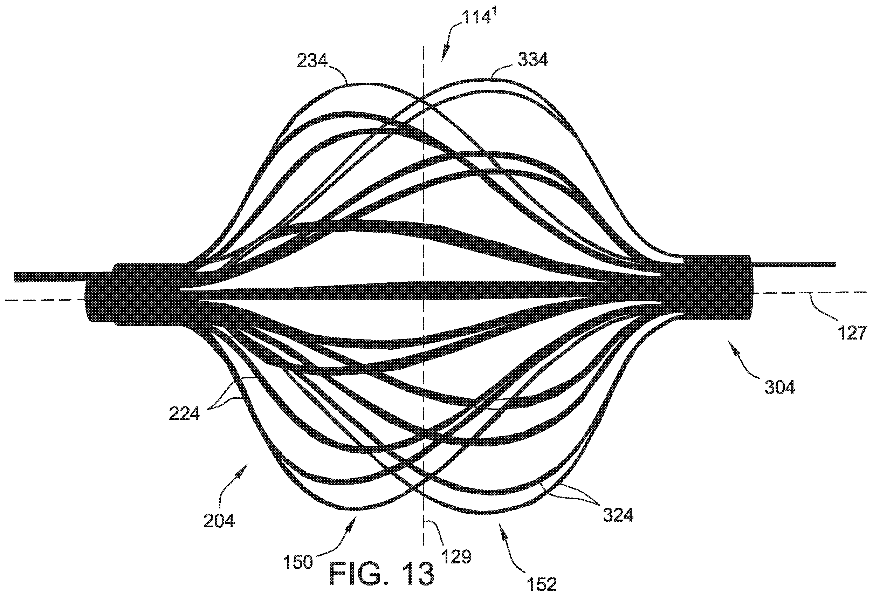

Figure 13 illustrates an alternative embodiment of an electrode assembly, designated using the reference numeral 114'. Electrode assembly 114' is symmetrical about longitudinal axis 127 and is asymmetrical about a plane 129 bisecting electrode assembly 114'. Maxima 234 and 334 are not located at the same longitudinal or axial position. Rather, maxima 234 and 334 are offset to define two maxima 150 and 152 of electrode assembly 114'. Electrode assembly 114' may generate a 360° circumferential ablation pattern between maximum 150 and maximum 152. Such a configuration may be suitable for ablation within a cylindrical vessel.

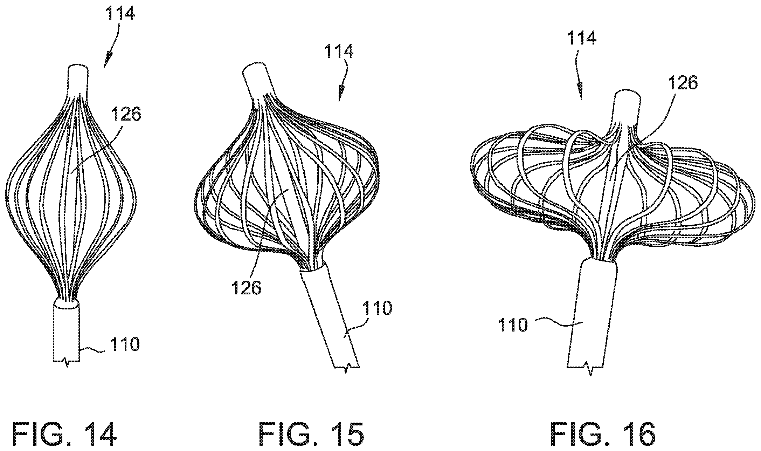

With reference to Figures 10 and 14-16, electrode assembly 114 may further include pull wire 126 coupled to distal end 120 thereof (e.g., coupled to distal end 210 of inner basket 204 and/or distal end 310 of outer basket 304). Pull wire 126 may be used to transition electrode assembly 114 from its collapsed configuration to its expanded configuration, or to vary the electrode assembly 114 between degrees of expansion within the expanded configuration. For example, as shown in Figures 14-16, electrode assembly 114 is maneuvered to its position within the patient's vasculature and advanced from catheter shaft 110 in a collapsed configuration. Pull wire 126 may be manipulated (e.g., using an actuator within handle 104, shown in Figure 1) to transition electrode assembly 114 between the configurations shown in Figures 14-16.

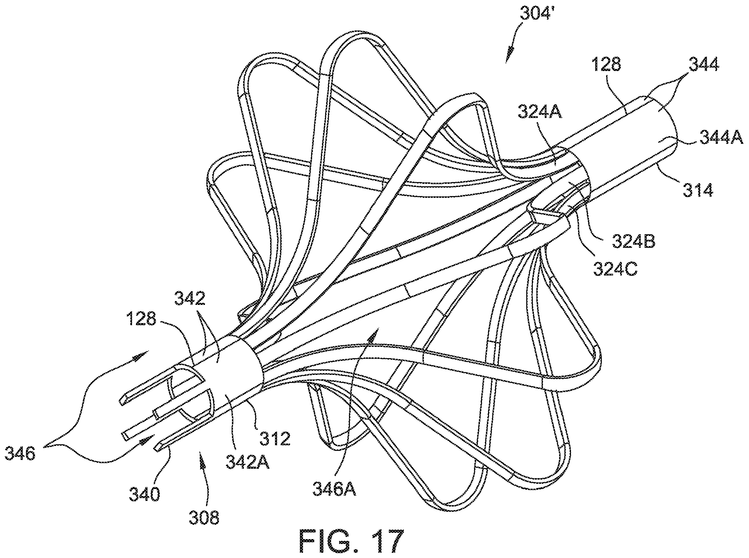

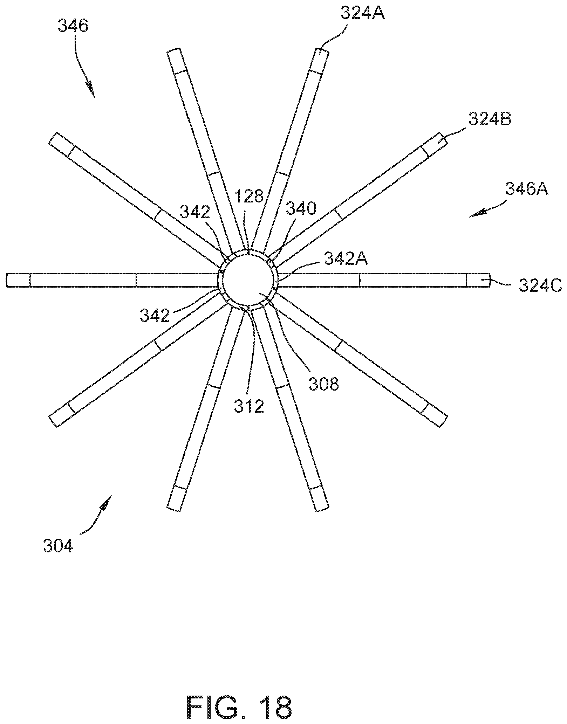

Figures 17 and 18 illustrate another alternative embodiment of an outer basket, designated using the reference number 304'. Outer basket 304' includes four tabs 340 extending from proximal end 308 thereof. In the illustrated embodiment, annular proximal end portion or collar 312 is separated or divided into sections 342. Each section 342 has a respective tab 340 extending therefrom. Although annular proximal end portion 312 includes four sections 342, annular proximal end portion 312 may include more than or fewer than four section 342. Likewise, annular distal end portion or collar 314 is separated or divided into sections 344. Although annular distal end portion 314 includes four sections 344, annular distal end portion 314 may include more or fewer than four section 344.

Sections 342 are electrically insulated from each adjacent section 342 by insulating material 128. Likewise, sections 344 are electrically insulated from each adjacent section by insulating material 128. Each section 344 corresponds to a section 342. Each strut 324 extends from a section 342 to a corresponding section 344. Each pair of corresponding sections 342-344 and the struts 324 extending therebetween define a "quadrant" 346 of outer basket 304'. For example, a first quadrant 346A of outer basket 304' includes a first (proximal) section 342A, a first (distal) section 344A, and struts 324A, 324B, and 324C extending between first section 342A and section 344A. In the illustrated embodiment, each tab 340 corresponds to a respective quadrant 346. As such, each quadrant 346 can be separately energized, as each quadrant 346 is insulated from each adjacent quadrant (e.g., by insulating material 128 at end portions 312, 314).

In some embodiments, an exemplary electrode assembly includes outer basket 304' divided into quadrants 346, and an inner basket divided into quadrants (not shown) in the same manner. In such embodiments, individual quadrants of the inner and outer basket may each be energized with a positive or negative charge, or individual quadrants may not be energized. This electrode assembly may be used during an ablation procedure to generate a 360° circumferential ablation pattern formed from quadrants energized with alternating charges, or to generate an ablation pattern over less than the entire circumference of the electrode assembly, such as two discrete ablation patterns of less than 180°.

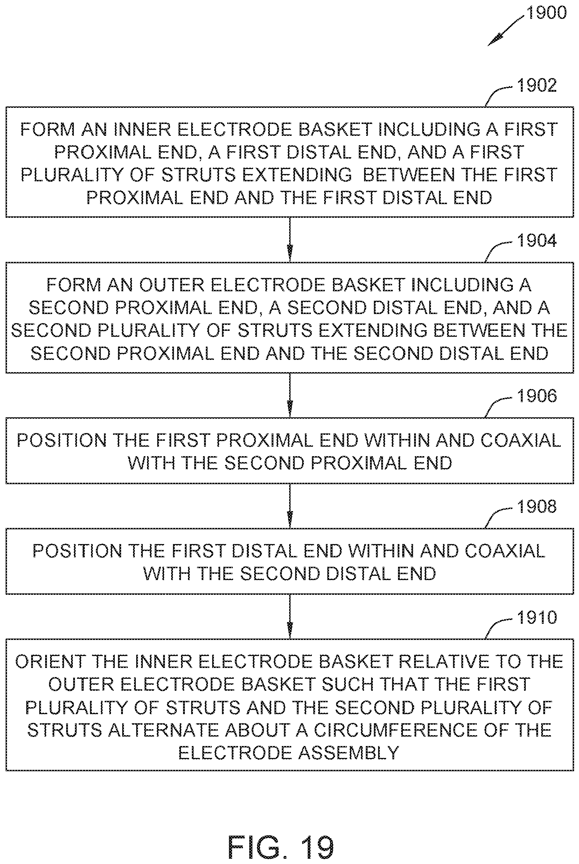

Figure 19 is a simplified process diagram for a method 1900 of forming an electrode assembly (e.g., electrode assembly 114, shown, for example, in Figures 1-4). Method 1900 includes forming 1902 an inner electrode basket (e.g., inner electrode basket 204) including a first proximal end (e.g., proximal end 208), a first distal end (e.g., distal end 210), and a first plurality of struts (e.g., struts 224, all shown in Figure 5) extending between the first proximal end and the first distal end.

Method 1900 also includes forming 1904 an outer electrode basket (e.g., outer electrode basket 304) including a second proximal end (e.g., proximal end 308), a second distal end (e.g., distal end 310), and a second plurality of struts (e.g., struts 324, all shown in Figure 6) extending between the second proximal end and the second distal end.

Method 1900 further includes positioning 1906 the first proximal end within and coaxial with the second proximal end, positioning 1908 the first distal end within and coaxial with the second distal end, and orienting 1910 the inner electrode basket relative to the outer electrode basket such that the first plurality of struts and the second plurality of struts alternate about a circumference of the electrode assembly.

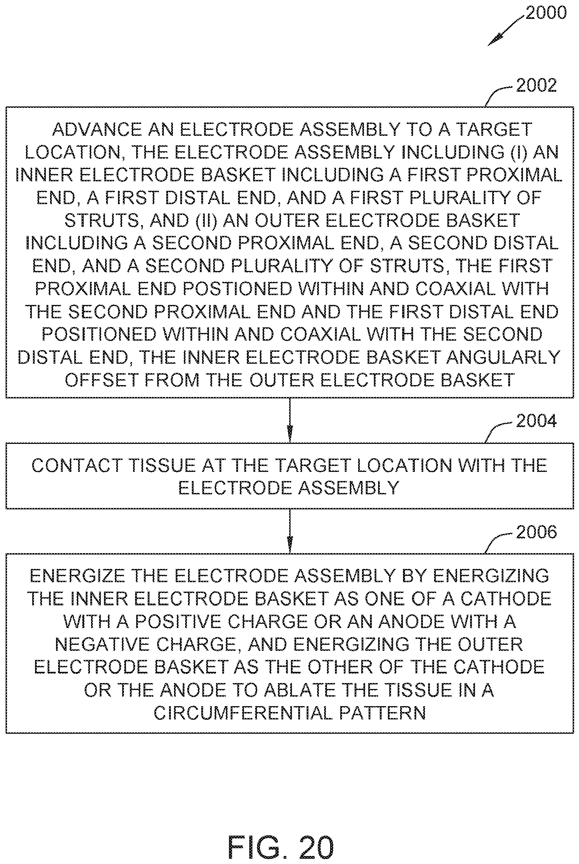

Figure 20 is a simplified process diagram for a non-claimed method 2000 of performing an ablation procedure using an electrode assembly (e.g., electrode assembly 114, shown, for example, in Figures 1-4). Method 2000 includes advancing 2002 the electrode assembly to a target location. In the exemplary embodiment, the electrode assembly includes (i) an inner electrode basket (e.g., inner electrode basket 204) including a first proximal end (e.g., proximal end 208), a first distal end (e.g., distal end 210), and a first plurality of struts (e.g., struts 224, all shown in Figure 5) extending between the first proximal end and the first distal end, and (ii) an outer electrode basket (e.g., outer electrode basket 304) including a second proximal end (e.g., proximal end 308), a second distal end (e.g., distal end 310), and a second plurality of struts (e.g., struts 324, all shown in Figure 6) extending between the second proximal end and the second distal end. Additionally, the first proximal end is positioned within and coaxial with the second proximal end and the first distal end is positioned within and coaxial with the second distal end such that the inner electrode basket is positioned within and coaxial with the outer electrode basket, and the inner electrode basket is angularly offset from the outer electrode basket such that the first plurality of struts and the second plurality of struts alternate about a circumference of the electrode assembly.

Method 2000 also includes contacting 2004 tissue (e.g., tissue 115, shown in Figure 9) at the target location with the electrode assembly, and energizing 2006 the electrode assembly (e.g., using a power supply such as generator 108, shown in Figure 1) by energizing the inner electrode basket as one of a cathode with a positive charge or an anode with a negative charge, and energizing the outer electrode basket as the other of the cathode or the anode, such that the first plurality of struts and the second plurality of struts alternate charge about the circumference of the electrode assembly and ablate the tissue in a circumferential pattern.

Although certain embodiments of this disclosure have been described above with a certain degree of particularity, those skilled in the art could make numerous alterations to the disclosed embodiments within the scope of the appended claims. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the scope of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

## Claims

1. An electrode assembly (114) for a catheter system, the electrode assembly comprising:
an inner electrode basket (204) comprising a first proximal end (208), a first distal end (210), and a first plurality of struts (224) extending between the first proximal end (208) and the first distal end (210); and
an outer electrode basket (304) comprising a second proximal end (308), a second distal end (310), and a second plurality of struts (324) extending between the second proximal end (308) and the second distal end (310),
wherein the first proximal end (208) is positioned within and is coaxial with the second proximal end (308) and the first distal end (210) is positioned within and coaxial with the second distal end (310) such that the inner electrode basket (204) is positioned within and coaxial with the outer electrode basket (304),
wherein the inner electrode basket (204) is angularly offset from the outer electrode basket (304) such that the first plurality of struts (224) and the second plurality of struts (324) alternate about a circumference of the electrode assembly (114), and
**characterized in that**
each strut of the first plurality of struts (224) and each strut of the second plurality of struts (324) defines a respective electrode along a respective length thereof.

2. The electrode assembly (114) claim 1, wherein, when energized, the inner electrode basket (204) is configured to act as one of a cathode with a positive charge or an anode with a negative charge, and the outer electrode basket (304) is configured to act as the other of the cathode or the anode.

3. The electrode assembly (114) of claim 2, further comprising an electrically insulating material disposed between the first proximal end (208) and the second proximal end (308) and between the first distal end (210) and the second distal end (310), such that the inner electrode basket (204) is electrically insulated from the outer electrode basket (304).

4. The electrode assembly (114) of claim 2, wherein, when energized, the first plurality of struts (224) has one of a positive charge or a negative charge, and the second plurality of struts (324) has the other of the positive charge or the negative charge, such that the electric charge of adjacent struts (224, 324) in the electrode assembly (114) alternates between a positive charge and a negative charge about the circumference of the electrode assembly (114), wherein an insulating material is preferably deposited on proximal ends of the first plurality of struts (224) and proximal ends of the second plurality of struts (324).

5. The electrode assembly of any one of claims 1 to 4, wherein the inner electrode basket (204) and the outer electrode basket (304) comprise an electrically conductive shape memory material and/or wherein each strut of the first plurality of struts (224) is equidistant from an adjacent one of the second plurality of struts (324).

6. The electrode assembly (114) of any one of claims 1 to 5, wherein each strut of the first plurality of struts (224) defines a first radial maximum at a first axial position between the first proximal end (208) and the first distal end (210), wherein the first radial maxima are circumferentially aligned with one another, and wherein each strut of the second plurality of struts (324) defines a second radial maximum at a second axial position between the second proximal end (308) and the second distal end (310), wherein the second radial maxima are circumferentially aligned with one another, wherein the first axial position is preferably the same as the second axial position.

7. The electrode assembly (114) of any one of claims 1 to 6, wherein the first proximal end (208) comprises a first annular proximal collar (212), the first distal end (210) comprises a first annular distal collar (214), the second proximal end comprises a second annular proximal collar (312), and the second distal end comprises a second annular distal collar (314).

8. The electrode assembly (114) of any one of claims 1 to 7, wherein the first proximal end (208) is divided into four proximal sections, each proximal section insulated from each adjacent proximal section, wherein the first distal end (210) is divided into four distal sections, each distal section insulated from each adjacent distal section, and wherein each distal section corresponds to a respective proximal section to form a proximal-distal section pair, and wherein a respective subset of the first plurality of struts (224) extends between each proximal-distal section pair to define a respective quadrant of the inner basket, wherein each quadrant is configured to be individually energized.

9. The electrode (114) assembly of any one of claims 1 to 8, wherein the first plurality of struts (224), the first distal end (210), and the first proximal end (208) are integrally formed, and wherein the second plurality of struts (324), the second distal end (310), and the second proximal end (308) are integrally formed.

10. A method of forming an electrode assembly, the method comprising:
forming an inner electrode basket (204) including a first proximal end (208), a first distal end (210), and a first plurality of struts (224) extending between the first proximal end (208) and the first distal end (210);
forming an outer electrode basket (304) including a second proximal end (308), a second distal end (310), and a second plurality of struts (324) extending between the second proximal end (308) and the second distal end (310), wherein each strut of the first plurality of struts (224) and each strut of the second plurality of struts (324) defines a respective electrode along a respective length thereof;
positioning the first proximal end (208) within and coaxial with the second proximal end (308);
positioning the first distal end (210) within and coaxial with the second distal end (310); and
orienting the inner electrode basket (204) relative to the outer electrode basket (304) such that the first plurality of struts (224) and the second plurality of struts (324) alternate about a circumference of the electrode assembly (114).

11. The method of claim 10, wherein forming the inner electrode basket comprises forming the inner electrode basket from an electrically conductive shape memory material, and wherein forming the outer electrode basket comprises forming the outer electrode basket from the electrically conductive shape memory material.

12. The method of claim 11, further comprising electrically insulating the inner electrode basket from the outer electrode basket, wherein electrically insulating the inner electrode basket from the outer electrode basket preferably comprises depositing an electrically insulating material between the first proximal end (208) and the second proximal end and between the first distal end (210) and the second distal end.

13. The method of claim 10, wherein forming the inner electrode basket further comprises heat-setting the inner electrode basket to define a profile of each strut of the first plurality of struts having a first radial maximum, wherein forming the outer electrode basket further comprises heat-setting the outer electrode basket to define a profile of each strut of the second plurality of struts having a second radial maximum, and wherein orienting the inner electrode basket relative to the outer electrode basket comprises orienting the inner electrode basket relative to the outer electrode basket such that the first radial maximum of each strut of the first plurality of struts is equidistant from an adjacent second maximum of a corresponding one of the second plurality of struts.

14. A catheter system comprising:
a flexible catheter shaft;
a handle coupled to a proximal end of the catheter shaft; and
the electrode assembly of any one of claims 1 to 9 and being sized for advancement through the catheter shaft to a distal end of the catheter shaft.

15. The catheter system of claim 14, further comprising a generator electrically coupled to the electrode assembly and configured to energize the electrode assembly, wherein, when the electrode assembly is energized by the generator, the inner electrode basket is configured to act as one of a cathode with a positive charge or an anode with a negative charge, and the outer electrode basket is configured to act as the other of the cathode or the anode.

## Patentansprüche

1. Elektrodenbaugruppe (114) für ein Kathetersystem, wobei die Elektrodenbaugruppe enthält:
einen inneren Elektrodenkorb (204), der ein erstes nahes Ende (208), ein erstes entferntes Ende (210) und eine Vielzahl von Streben (224) aufweist, die sich zwischen dem ersten nahen Ende (208) und dem ersten entfernten Ende (210) erstrecken; und
einen äußeren Elektrodenkorb (304), der ein zweites nahes Ende (308), ein zweites entferntes Ende (310) und eine zweite Vielzahl von Streben (324) aufweist, die sich zwischen dem zweiten nahen Ende (308) und dem zweiten entfernten Ende (310) erstrecken,
wobei das erste nahe Ende (208) innerhalb des zweiten nahen Endes (308) positioniert ist und koaxial zu dem zweiten nahen Ende (308) ist und das erste entfernte Ende (210) innerhalb des zweiten entfernten Endes (310) positioniert ist und koaxial zu dem zweiten entfernten Ende (310) ist, so dass der innere Elektrodenkorb (204) innerhalb des äußeren Elektrodenkorbs (304) positioniert ist und koaxial zu dem äußeren Elektrodenkorb (304) ist,
wobei der innere Elektrodenkorb (204) von dem äußeren Elektrodenkorb (304) winklig versetzt ist, so dass sich die erste Vielzahl von Streben (224) und die zweite Vielzahl von Streben (324) über einen Umfang der Elektrodenbaugruppe (114) abwechseln, und
**dadurch gekennzeichnet, dass**
jede Strebe aus der ersten Vielzahl von Streben (224) und jede Strebe aus der zweiten Vielzahl von Streben (324) eine entsprechende Elektrode darauf aufweist.

2. Elektrodenbaugruppe (114) nach Anspruch 1, wobei, wenn mit Energie beaufschlagt, der innere Elektrodenkorb (204) zum Wirken als eine aus einer Kathode mit einer positiven Ladung oder einer Anode mit einer negativen Ladung ausgebildet ist, und der äußere Elektrodenkorb (304) zum Wirken als die andere aus der Kathode oder der Anode ausgebildet ist.

3. Elektrodenbaugruppe (114) nach Anspruch 2, ferner enthaltend ein elektrisch isolierendes Material, das zwischen dem ersten nahen Ende (208) und dem zweiten nahen Ende (308) und zwischen dem ersten entfernten Ende (210) und dem zweiten entfernten Ende (310) angeordnet ist, so dass der innere Elektrodenkorb (204) von dem äußeren Elektrodenkorb (304) elektrisch isoliert ist.

4. Elektrodenbaugruppe (114) nach Anspruch 2, wobei, wenn mit Energie beaufschlagt, die erste Vielzahl von Streben (224) eine aus einer positiven Ladung oder einer negativen Ladung aufweist, und die zweite Vielzahl von Streben (324) die andere aus der positiven Ladung oder der negativen Ladung aufweist, so dass die elektrische Ladung benachbarter Streben (224, 324) in der Elektrodenbaugruppe (114) zwischen einer positiven Ladung und einer negativen Ladung über den Umfang der Elektrodenbaugruppe (114) wechselt, wobei ein isolierendes Material vorzugsweise auf nahen Enden der ersten Vielzahl von Streben (224) und nahen Enden der zweiten Vielzahl von Streben (324) angeordnet ist.

5. Elektrodenbaugruppe nach einem der Ansprüche 1 bis 4, wobei der innere Elektrodenkorb (204) und der äußere Elektrodenkorb (304) ein elektrisch leitfähiges Formgedächtnismaterial aufweist und/oder wobei jede Strebe aus der ersten Vielzahl von Streben (224) von einer benachbarten einen aus der zweiten Vielzahl von Streben (324) gleichmäßig beabstandet ist.

6. Elektrodenbaugruppe (114) nach einem der Ansprüche 1 bis 5, wobei jede Strebe aus der ersten Vielzahl von Streben (224) ein erstes radiales Maximum an einer ersten axialen Position zwischen dem ersten nahen Ende (208) und dem ersten entfernten Ende (210) definiert, wobei die ersten radialen Maxima über den Umfang zueinander ausgerichtet sind, und wobei jede Strebe aus der zweiten Vielzahl von Streben (324) ein zweites radiales Maximum an einer zweiten axialen Position zwischen dem zweiten nahen Ende (308) und dem zweiten entfernten Ende (310) definiert, wobei die zweiten radialen Maxima über dem Umfang zueinander ausgerichtet sind, wobei die erste axiale Position vorzugsweise dieselbe ist wie die zweite axiale Position.

7. Elektrodenbaugruppe (114) nach einem der Ansprüche 1 bis 6, wobei das erste nahe Ende (208) einen ersten ringförmigen nahen Bund (212) aufweist, das erste entfernte Ende (210) einen ersten ringförmigen entfernten Bund (212) aufweist, das zweite nahe Ende einen zweiten ringförmigen nahen Bund (312) aufweist und das zweite entfernte Ende einen zweiten ringförmigen entfernten Bund (314) aufweist.

8. Elektrodenbaugruppe (114) nach einem der Ansprüche 1 bis 7, wobei das erste nahe Ende (208) in vier nahe Abschnitte unterteilt ist, wobei jeder nahe Abschnitt von jedem benachbarten nahen Abschnitt isoliert ist, wobei das erste entfernte Ende (210) in vier entfernte Abschnitte unterteilt ist, wobei jeder entfernte Abschnitt von jedem benachbarten entfernten Abschnitt isoliert ist, und wobei jeder entfernte Abschnitt einem entsprechenden nahen Abschnitt entspricht zum Bilden eines Nah-Fern-Abschnittspaars, und wobei eine entsprechende Untermenge aus der ersten Vielzahl von Streben (224) sich zwischen jedem Nah-Fern-Abschnittspaar zum Definieren eines entsprechenden Quadranten des inneren Korbs erstreckt, wobei jeder Quadrant derart ausgebildet ist, dass er individuell mit Energie beaufschlagt wird.

9. Elektrodenbaugruppe (114) nach einem der Ansprüche 1 bis 8, wobei die erste Vielzahl von Streben (224), das erste entfernte ende (210) und das erste nahe Ende (208) integral ausgebildet sind, und wobei die zweite Vielzahl von Streben (324), das zweite entfernte Ende (310) und das zweite nahe Ende (308) integral ausgebildet sind.

10. Verfahren zum Bilden einer Elektrodenbaugruppe, wobei das Verfahren enthält:
Bilden eines inneren Elektrodenkorbs (204), der ein erstes nahes Ende (208), ein erstes entferntes Ende (210) und eine erste Vielzahl von Streben (224) aufweist, die sich zwischen dem ersten nahen Ende (208) und dem ersten entfernten Ende (210) erstrecken;
Bilden eines äußeren Elektrodenkorbs (304), der ein zweites nahes Ende (308), ein zweites entferntes Ende (310) und eine zweite Vielzahl von Streben (324) aufweist, die sich zwischen dem zweiten nahen Ende (308) und dem zweiten entfernten Ende (310) erstrecken, wobei jede Strebe aus der ersten Vielzahl von Streben (224) und jede Strebe aus der zweiten Vielzahl von Streben (324) eine entsprechende Elektrode darauf aufweisen;
Positionieren des ersten nahen Endes (208) innerhalb des zweiten nahen Endes (308) und koaxial zu dem zweiten nahen Ende (308);
Positionieren des ersten entfernten Endes (210) innerhalb des zweiten entfernten Endes (310) und koaxial zu dem zweiten entfernten Ende (310); und
Ausrichten des inneren Elektrodenkorbs (204) relativ zu dem äußeren Elektrodenkorb (304), so dass sich die erste Vielzahl von Streben (224) und die zweite Vielzahl von Streben (324) über einen Umfang der Elektrodenbaugruppe (114) abwechseln.

11. Verfahren nach Anspruch 10, wobei das Bilden des inneren Elektrodenkorbs ein Bilden des inneren Elektrodenkorbs aus einem elektrisch leitfähigen Formgedächtnismaterial enthält, und wobei das Bilden des äußeren Elektrodenkorbs ein Bilden des äußeren Elektrodenkorbs aus dem elektrisch leitfähigen Formgedächtnismaterial enthält.

12. Verfahren nach Anspruch 11, ferner enthaltend ein elektrisches Isolieren des inneren Elektrodenkorbs von dem äußeren Elektrodenkorb, wobei das elektrische Isolieren des inneren Elektrodenkorbs von dem äußeren Elektrodenkorb vorzugsweise ein Anordnen eines elektrisch isolierenden Materials zwischen dem ersten nahen Ende (208) und dem zweiten nahen Ende und zwischen dem ersten entfernten Ende (210) und dem zweiten entfernten Ende enthält.

13. Verfahren nach Anspruch 10, wobei das Bilden des inneren Elektrodenkorbs ferner ein Thermofixieren des inneren Elektrodenkorbs zum Definieren eines Profils jeder Strebe aus der ersten Vielzahl von Streben mit einem ersten radialen Maximum enthält, wobei das Bilden des äußeren Elektrodenkorbs ferner ein Thermofixieren des äußeren Elektrodenkorbs zum Definieren eines Profils jeder Strebe aus der zweiten Vielzahl von Streben mit einem zweiten radialen Maximum enthält, und wobei das Ausrichten des inneren Elektrodenkorbs relativ zu dem äußeren Elektrodenkorb ein Ausrichten des inneren Elektrodenkorbs relativ zu dem äußeren Elektrodenkorb derart enthält, dass das erste radiale Maximum jeder Strebe aus der ersten Vielzahl von Streben von einem benachbarten zweiten Maximum einer entsprechenden einen aus der zweiten Vielzahl von Streben gleichmäßig beabstandet ist.

14. Kathetersystem, enthaltend:
einen flexiblen Katheterschaft;
einen Handgriff, der mit einem nahen Ende des Katheterschafts gekoppelt ist; und
die Elektrodenbaugruppe nach einem der Ansprüche 1 bis 9 und die zum Befördern durch den Katheterschaft zu einem entfernten Ende des Katheterschafts ausgelegt ist.

15. Kathetersystem nach Anspruch 14, ferner enthaltend einen Generator, der mit der Elektrodenbaugruppe elektrisch gekoppelt ist und dazu ausgebildet ist, die Elektrodenbaugruppe mit Energie zu beaufschlagen, wobei, wenn die Elektrodenbaugruppe durch den Generator mit Energie beaufschlagt wird, der innere Elektrodenkorb zum Wirken als eine aus einer Kathode mit einer positiven Ladung oder einer Anode mit einer negativen Ladung ausgebildet ist und der andere Elektrodenkorb zum Wirken als die andere aus der Kathode oder der Anode ausgebildet ist.

## Revendications

1. Ensemble d'électrode (114) pour un système de cathéter, l'ensemble d'électrode comprenant :
un panier d'électrode interne (204) comprenant une première extrémité proximale (208), une première extrémité distale (210), et une première pluralité d'entretoises (224) s'étendant entre la première extrémité proximale (208) et la première extrémité distale (210) ; et
un panier d'électrode externe (304) comprenant une deuxième extrémité proximale (308), une deuxième extrémité distale (310), et une deuxième pluralité d'entretoises (324) s'étendant entre la deuxième extrémité proximale (308) et la deuxième extrémité distale (310),
dans lequel la première extrémité proximale (208) est positionnée à l'intérieur de et de manière coaxiale avec la deuxième extrémité proximale (308), et la première extrémité distale (210) est positionnée à l'intérieur de et de manière coaxiale avec la deuxième extrémité distale (310), de sorte que le panier d'électrode interne (204) est positionné à l'intérieur et coaxial vis-à-vis du panier d'électrode externe (304),
dans lequel le panier d'électrode interne (204) est décalé de manière angulaire vis-à-vis du panier d'électrode externe (304) de sorte que la première pluralité d'entretoises (224) et la deuxième pluralité d'entretoises (324) alternent autour d'une circonférence de l'ensemble d'électrode (114), et
**caractérisé en ce que**
chaque entretoise de la première pluralité d'entretoises (224) et chaque entretoise de la deuxième pluralité d'entretoises (324) comprend une électrode respective sur celle-ci.

2. Ensemble d'électrode (114) selon la revendication 1, dans lequel, lorsqu'il est mis sous tension, le panier d'électrode interne (204) est configuré pour agir comme un élément parmi une cathode avec une charge positive ou une anode avec une charge négative, et le panier d'électrode externe (304) est configuré pour agir comme l'autre élément parmi la cathode ou l'anode.

3. Ensemble d'électrode (114) selon la revendication 2, comprenant en outre un matériau électriquement isolant disposé entre la première extrémité proximale (208) et la deuxième extrémité proximale (308) et entre la première extrémité distale (210) et la deuxième extrémité distale (310), de sorte que le panier d'électrode interne (204) est électriquement isolé du panier d'électrode externe (304).

4. Ensemble d'électrode (114) selon la revendication 2, dans lequel, lorsqu'il est alimenté, la première pluralité d'entretoises (224) a une charge parmi une charge positive ou une charge négative, et la deuxième pluralité d'entretoises (324) a l'autre charge parmi la charge positive ou la charge négative, de sorte que la charge électrique des entretoises adjacentes (224, 324) dans l'ensemble d'électrode (114) alterne entre une charge positive et une charge négative autour de la circonférence de l'ensemble d'électrode (114), dans lequel un matériau isolant est de préférence déposé sur les extrémités proximales de la première pluralité d'entretoises (224) et les extrémités proximales de la deuxième pluralité d'entretoises (324).

5. Ensemble d'électrode selon l'une quelconque des revendications 1 à 4, dans lequel le panier d'électrode interne (204) et le panier d'électrode externe (304) comprennent un matériau à mémoire de forme électriquement conducteur et/ou dans lequel chaque entretoise de la première pluralité d'entretoises (224) est équidistante d'une entretoise adjacente de la deuxième pluralité d'entretoises (324).

6. Ensemble d'électrode (114) selon l'une quelconque des revendications 1 à 5, dans lequel chaque entretoise de la première pluralité d'entretoises (224) définit un premier maximum radial à une première position axiale entre la première extrémité proximale (208) et la première extrémité distale (210), dans lequel les premiers maxima radiaux sont alignés de manière circonférentielle les uns avec les autres, et dans lequel chaque entretoise de la deuxième pluralité d'entretoises (324) définit un deuxième maximum radial à une deuxième position axiale entre la deuxième extrémité proximale (308) et la deuxième extrémité distale (310), dans lequel les deuxièmes maxima radiaux sont alignés de manière circonférentielle les uns avec les autres, dans lequel la première position axiale est de préférence la même que la deuxième position axiale.

7. Ensemble d'électrode (114) selon l'une quelconque des revendications 1 à 6, dans lequel la première extrémité proximale (208) comprend un premier collier proximal annulaire (212), la première extrémité distale (210) comprend un premier collier distal annulaire (214), la deuxième extrémité proximale comprend un deuxième collier proximal annulaire (312), et la deuxième extrémité distale comprend un deuxième collier distal annulaire (314).

8. Ensemble d'électrode (114) selon l'une quelconque des revendications 1 à 7, dans lequel la première extrémité proximale (208) est divisée en quatre sections proximales, chaque section proximale étant isolée de chaque section proximale adjacente, dans lequel la première extrémité distale (210) est divisée en quatre sections distales, chaque section distale étant isolée de chaque section distale adjacente, et dans lequel chaque section distale correspond à une section proximale respective pour former une paire de sections proximale-distale, et dans lequel un sous-ensemble respectif de la première pluralité d'entretoises (224) s'étend entre chaque paire de sections proximale-distale pour définir un quadrant respectif du panier intérieur, dans lequel chaque quadrant est configuré pour être alimenté individuellement.

9. Ensemble d'électrode (114) selon l'une quelconque des revendications 1 à 8, dans lequel la première pluralité d'entretoises (224), la première extrémité distale (210) et la première extrémité proximale (208) sont formées d'un seul tenant, et dans lequel la deuxième pluralité d'entretoises (324), la deuxième extrémité distale (310) et la deuxième extrémité proximale (308) sont formées d'un seul tenant.

10. Procédé de formation d'un ensemble d'électrode, le procédé comprenant les étapes consistant à :
former un panier d'électrode interne (204) comprenant une première extrémité proximale (208), une première extrémité distale (210), et une première pluralité d'entretoises (224) s'étendant entre la première extrémité proximale (208) et la première extrémité distale (210) ;
former un panier d'électrode externe (304) comprenant une deuxième extrémité proximale (308), une deuxième extrémité distale (310), et une deuxième pluralité d'entretoises (324) s'étendant entre la deuxième extrémité proximale (308) et la deuxième extrémité distale (310), dans lequel chaque entretoise de la première pluralité d'entretoises (224) et chaque entretoise de la deuxième pluralité d'entretoises (324) comprend une électrode respective sur celle-ci ;
positionner la première extrémité proximale (208) à l'intérieur de et de manière coaxiale avec la deuxième extrémité proximale (308);
positionner la première extrémité distale (210) à l'intérieur de et de manière coaxiale avec la deuxième extrémité distale (310) ; et
orienter le panier d'électrode interne (204) par rapport au panier d'électrode externe (304) de sorte que la première pluralité d'entretoises (224) et la deuxième pluralité d'entretoises (324) alternent autour d'une circonférence de l'ensemble d'électrode (114).

11. Procédé selon la revendication 10, dans lequel la formation du panier d'électrode interne comprend la formation du panier d'électrode interne à partir d'un matériau à mémoire de forme électriquement conducteur, et dans lequel la formation du panier d'électrode externe comprend la formation du panier d'électrode externe à partir du matériau à mémoire de forme électriquement conducteur.

12. Procédé selon la revendication 11, comprenant en outre l'isolation électrique du panier d'électrode interne du panier d'électrode externe, dans lequel l'isolation électrique du panier d'électrode interne du panier d'électrode externe comprend de préférence le dépôt d'un matériau électriquement isolant entre la première extrémité proximale (208) et la deuxième extrémité proximale et entre la première extrémité distale (210) et la deuxième extrémité distale.

13. Procédé selon la revendication 10, dans lequel la formation du panier d'électrode interne comprend en outre le thermofixage du panier d'électrode interne pour définir un profil de chaque entretoise de la première pluralité d'entretoises ayant un premier maximum radial, dans lequel la formation du panier d'électrode externe comprend en outre le thermofixage du panier d'électrode externe pour définir un profil de chaque entretoise de la deuxième pluralité d'entretoises ayant un deuxième maximum radial, et dans lequel l'orientation du panier d'électrode interne par rapport au panier d'électrode externe comprend l'orientation du panier d'électrode interne par rapport au panier d'électrode externe de sorte que le premier maximum radial de chaque entretoise de la première pluralité d'entretoises est équidistant d'un deuxième maximum adjacent d'une entretoise correspondante de la deuxième pluralité d'entretoises.

14. Système de cathéter comprenant :
une tige de cathéter flexible ;
une poignée couplée à une extrémité proximale de la tige de cathéter ; et
l'ensemble d'électrode selon l'une quelconque des revendications 1 à 9 et étant dimensionné pour une progression à travers la tige de cathéter jusqu'à une extrémité distale de la tige de cathéter.

15. Système de cathéter selon la revendication 14, comprenant en outre un générateur couplé électriquement à l'ensemble d'électrode et configuré pour alimenter l'ensemble d'électrode, dans lequel, lorsque l'ensemble d'électrode est alimenté par le générateur, le panier d'électrode interne est configuré pour agir comme une cathode avec une charge positive ou une anode avec une charge négative, et le panier d'électrode externe est configuré pour agir comme l'autre élément parmi la cathode ou l'anode.
